# FASCICULE DE BREVET EUROPEEN

(11) **EP 2 724 691 B1**
(45) Date de publication et mention de la délivrance du brevet: **17.06.2015**
(21) Numéro de dépôt: 13189565.8
(22) Date de dépôt: 21.10.2013
(51) Int. Cl.: A61F 2/30, A61F 2/40

(54) **Implant glénoïdien comprenant une cupule destinée à coopérer avec une tête humérale prothétique**
Glenoidales Implantat, das eine Kuppel für den Anschluss an eine Humeruskopfprothese umfasst
Glenoid implant including a cup intended for engaging with a prosthetic humeral head

(30) Priorité: 26.10.2012 FR 1260205
(43) Date de publication de la demande: 30.04.2014
(73) Titulaire: Aston Medical, 42000 Saint Etienne (FR)
(72) Inventeur: Baulot, Emmanuel, 21000 DIJON (FR); Lerue, Olivier, 59130 LAMBERSAERT (FR); Souquet, David, 06100 NICE (FR); Scarlat, Marius, 83400 HYERES (FR); Colombier, Michel, 34000 Montpellier (FR)
(74) Mandataire: Cabinet Laurent & Charras

(56) Documents cités:
- WO-A1-2010/076534
- DE-A1-102010 052 634
- FR-A1- 2 780 635
- US-A1- 2007 260 321

## Description

L'invention s'attache au secteur technique des implants orthopédiques et concerne, plus particulièrement, un implant glénoïdien pour l'arthroplastie de l'articulation de l'épaule.

De manière parfaitement connue pour un homme du métier, une prothèse glénoïdienne comprend une cupule en polyéthylène qui peut être soit directement fixée, par exemple au moyen de ciment, dans la cavité glénoïdienne, soit être accouplée à une embase métallique destinée à être fixée dans ladite cavité glénoïdienne.

Sur cette cupule en polyéthylène vient en appui, avec capacité d'articulation, une tête humérale prothétique.

Différentes solutions techniques ont été proposées.

L'invention concerne plus particulièrement un implant glénoïdien du type de celui défini dans le document WO 2010/076534. Il ressort de ce document que la cupule est de forme générale circulaire en étant conformée pour être fixée et encastrée dans l'épaisseur de la cavité anatomique glénoïdienne de sorte que la surface d'appui et de glissement de ladite cupule s'intègre dans la continuité de ladite cavité anatomique afin d'obtenir une congruence parfaite avec la tête humérale.

Ces caractéristiques permettent de remplacer le cartilage sans en modifier l'architecture biomécanique. En effet, on rappelle que des études biomécaniques ont démontré que le centre instantané de rotation se situe dans une surface de l'ordre de 5 à 8 mm de diamètre et, non pas, suivant une translation de rotation de l'articulation comme cela est généralement admis par les praticiens.

A partir de ces caractéristiques le problème que se propose de résoudre l'invention est d'améliorer le centrage de la cupule et d'obtenir un joint biologique et d'éviter au maximum l'effet d'usure du polyéthylène constituant ladite cupule.

Selon une caractéristique à la base de l'invention, la cupule présente en débordement de sa périphérie et seulement dans sa partie supérieure, des lamelles déformables de stabilisation coopérant avec le spongieux, la partie inférieure de la cupule étant lisse et accouplée à une embase métallique de forme circulaire, le bord périphérique de ladite partie lisse et de ladite embrase étant disposés en alignement en ayant le même diamètre.

Il résulte donc de ces caractéristiques que les lamelles situées en partie supérieure de la cupule constituent un joint biologique tandis que la partie inférieure de la cupule assure son centrage.

Avantageusement, les lamelles sont orientées en direction du haut de manière à favoriser l'implantation et l'ancrage après implantation.

Selon une autre caractéristique, la cupule présente en débordement de sa face de dessous, au moins un ergot anti rotatoire coopérant avec au moins une entaille formée dans l'épaisseur de l'embrase à partir de son bord périphérique.

Pour résoudre le problème posé d'améliorer le centrage de la cupule, la partie lisse de la cupule présente une section transversale dégressive en direction de l'embase en forme de tronc de cône.

Selon une autre caractéristique, la cupule présente, à partir de sa face d'appui avec l'embase, un chambrage présentant des formes de clipage aptes à coopérer avec des formes complémentaires que présente une partie formée en débordement de ladite embase.

Avantageusement, l'embase présente un plot d'encrage et de stabilité, lequel plot présente au moins une fente pour créer un effet de press-fit.

L'invention est exposée ci-après plus en détails à l'aide des figures des dessins annexés dans lesquels :

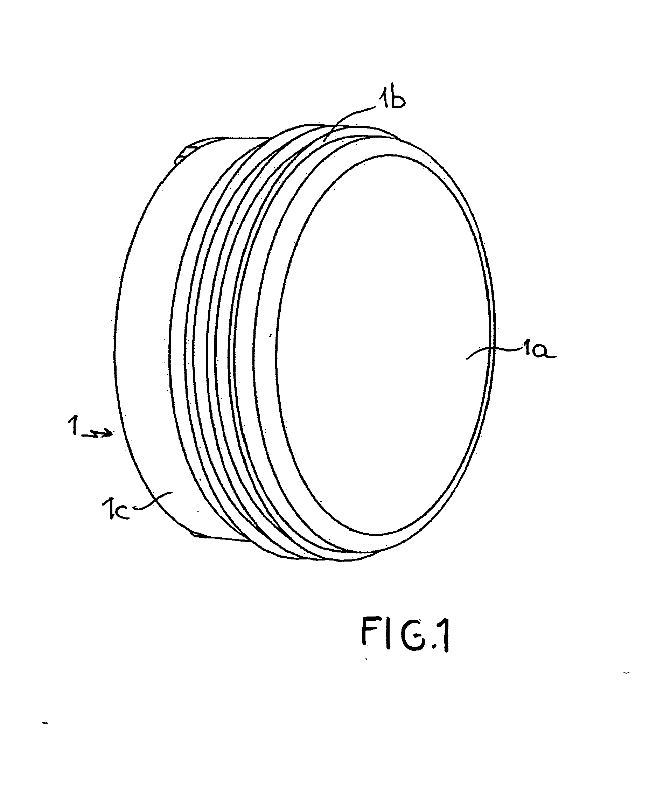

- la figure 1 est une vue en perspective de la cupule considérée en vue du dessus,

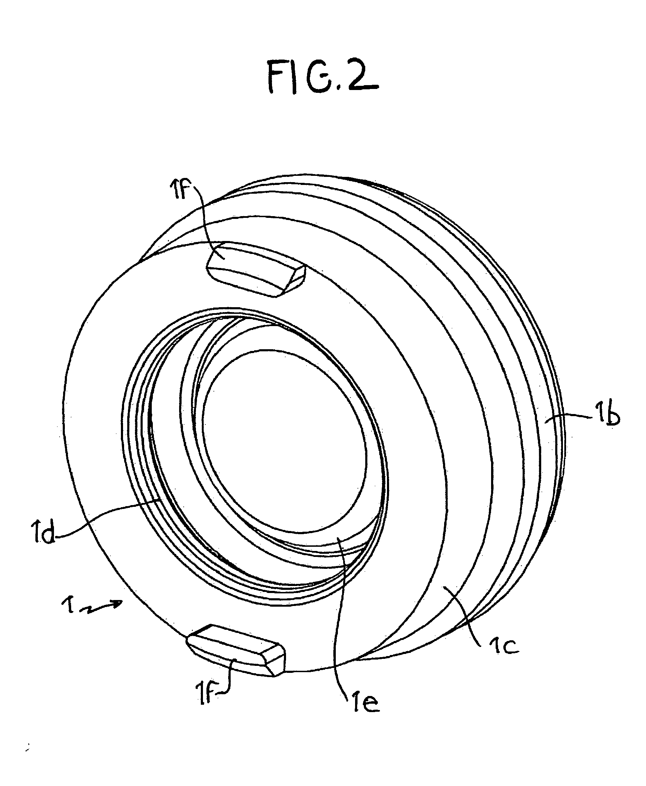

- la figure 2 est une vue en perspective de la cupule considérée en vue de dessous,

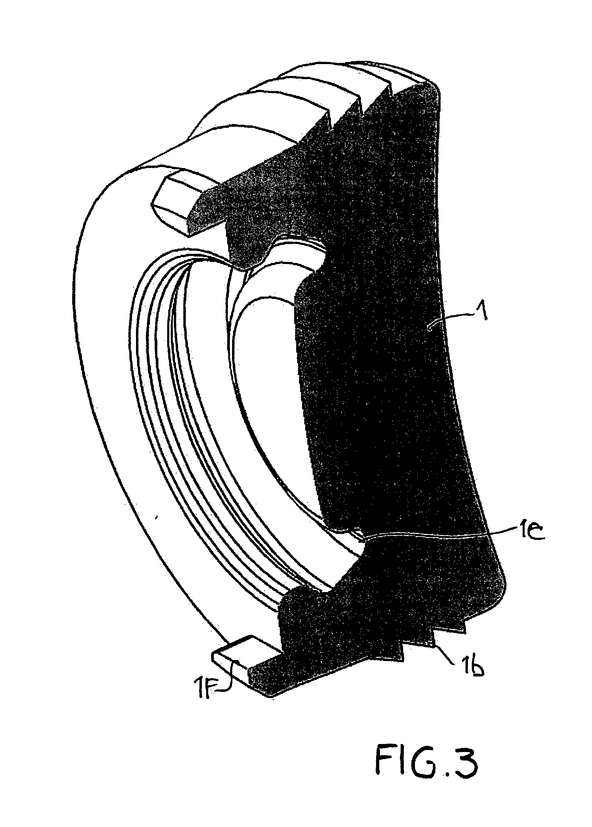

- la figure 3 est une vue en perspective de la cupule avec coupe partielle,

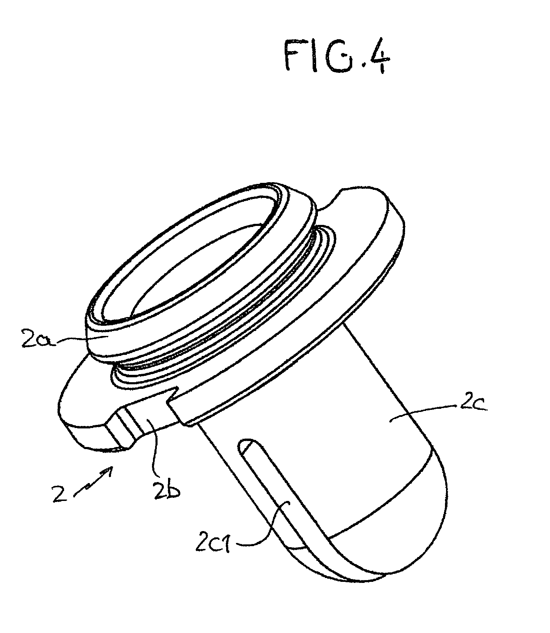

- la figure 4 est une vue en perspective de l'embase de fixation,

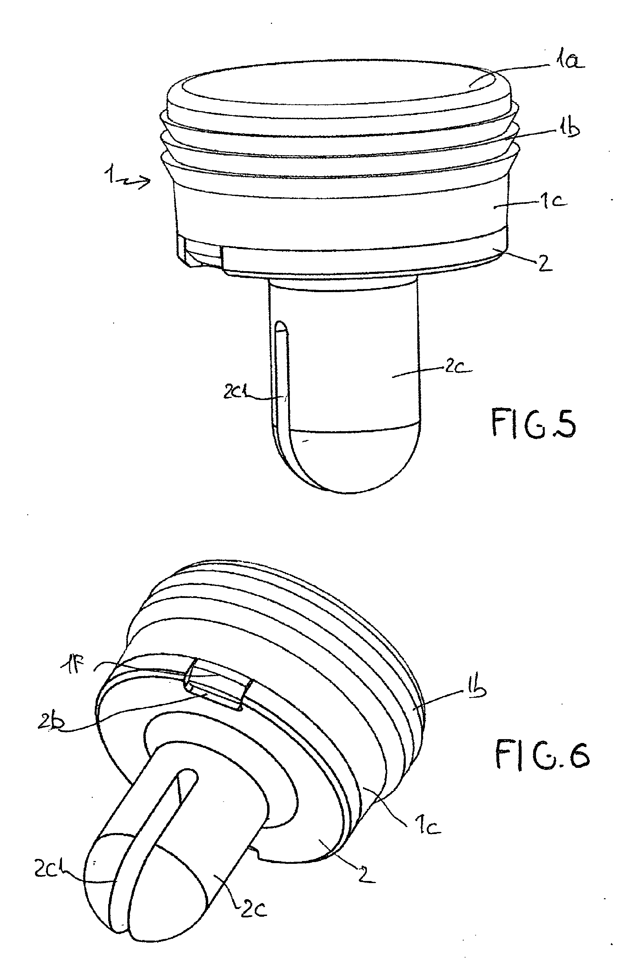

- la figure 5 est une vue en perspective de l'ensemble de l'implant glénoïdien montrant la cupule accouplée à l'embase,
- la figure 6 est une vue en perspective de la cupule considérée en vue du dessous de l'ensemble de l'implant glénoïdien,

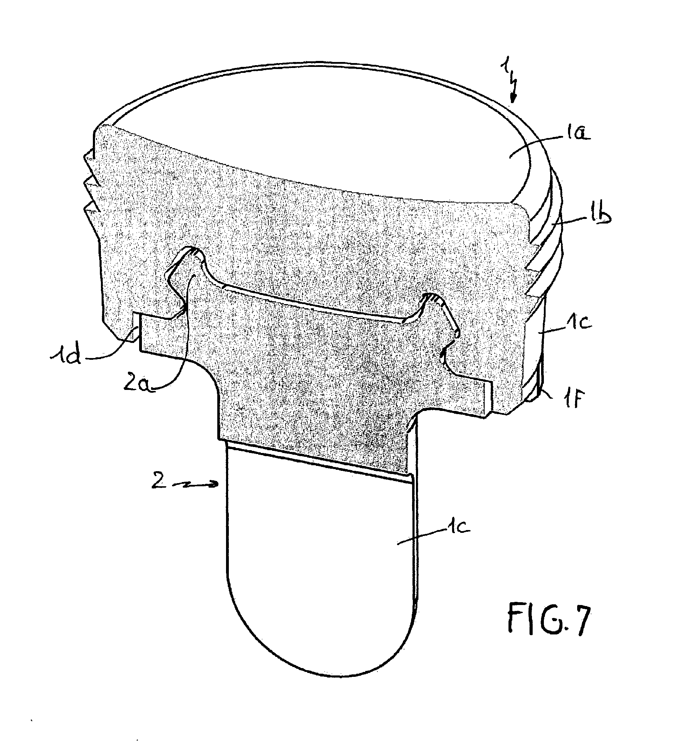

- la figure 7 est une vue en perspective et en coupe de l'ensemble de l'implant glénoïdien.

L'implant glénoïdien présente une cupule (1) en polyéthylène présentant, de manière connue, une surface d'appui et de glissement concave (1a) pour l'articulation d'une tête humérale prothétique non représentée.

Comme il ressort de l'enseignement du brevet WO 2010/076534, la cupule (1) de forme générale circulaire est destinée à être encastrée dans la cavité anatomique glénoïdienne de sorte que sa surface d'appui et de glissement (1a) s'intègre dans la continuité de la cavité anatomique afin d'obtenir une congruence parfaite avec la tête humérale.

Comme le montrent les figures des dessins, la partie supérieure de la cupule (1) présente, en débordement de sa périphérie, des lamelles déformables de stabilisation (1b) coopérant avec le spongieux. Ces lamelles (1b) sont orientées en direction du haut de manière à favoriser l'implantation de la cupule et d'améliorer l'ancrage après avoir implanté ladite cupule.

Ces lamelles (1b) font office de joint biologique. La partie inférieure (1c) de la cupule est lisse et est agencée pour être accouplée à une embase métallique (2) de forme générale circulaire.

A noter que le bord périphérique de la partie lisse (1c) de la cupule et le bord périphérique de l'embase (2), après accouplement, sont disposés en alignement, ledit bord périphérique ayant le même diamètre.

Avantageusement la portée lisse (1c) présente une section transversale dégressive en direction de l'embase en forme de tronc de cône afin de favoriser le centrage au moment de l'impaction.

La cupule (1) et l'embase (2) présentent des formes complémentaires d'accouplement.

La cupule (1) présente à partir de sa face d'appui avec l'embase (2), un chambrage (1d) présentant des formes de clipage aptes à coopérer avec des formes complémentaires que présentent une portée circulaire (2a) formée en débordement de l'embase (2).

Pour assurer une indexation angulaire de la cupule par rapport à l'embase, la cupule (1) présente en sa périphérie et en débordement de sa face de dessous, au moins un ergot anti-rotatoire (1F) coopérant avec au moins une entaille (2b) formée dans l'épaisseur de l'embase (2) à partir de son bord périphérique.

Avantageusement, la cupule (1) présente deux ergots diamétralement opposés (1f) coopérant avec deux entailles (2b).

Selon une autre caractéristique l'embase (2) présente un plot d'encrage et de stabilité (2c) de section cylindrique.

Ce plot présente au moins une fente (2c1) formée selon ses génératrices, pour créer un effet de press-fit au moment de l'impaction de l'embase (2).

Les avantages ressortent bien de la description, en particulier, on souligne et on rappelle, en plus de ceux relevant de la conception de base de l'implant glénoïdien tel que défini dans le document WO 2010/076534, la création d'un joint biologique au niveau de la cupule avec un centrage amélioré au niveau de l'impaction ainsi que la suppression d'éventuels débris de polyéthylène grâce à l'embase métallique qui affleure la zone circulaire lisse d'appui de ladite cupule.

## Revendications

1. Implant glénoïdien comprenant une cupule (1) destinée à coopérer avec une tête humérale prothétique, ladite cupule (1) est de forme générale circulaire et présente des agencements de positionnement et de fixation aptes à permettre son encastrement dans la cavité anatomique glénoïdienne, de sorte que la surface d'appui et de glissement (1a) de ladite cupule s'intègre dans la continuité de ladite cavité anatomique, pour obtenir une congruence parfaite avec la tête humérale **caractérisé en ce que** la cupule (1) présente, en débordement de sa périphérie et seulement dans sa partie supérieure, des lamelles déformables de stabilisation (1b) coopérant avec le spongieux, la partie inférieure (1c) de la cupule étant lisse et accouplée à une embase métallique de forme circulaire (2), le bord périphérique de ladite partie lisse (1c) et de ladite embase (2) étant disposés en alignement en ayant le même diamètre.

2. Dispositif selon la revendication 1 **caractérisé en ce que** les lamelles (1b) sont orientées en direction du haut de manière à favoriser l'implantation et l'ancrage après implantation.

3. Dispositif selon l'une quelconque des revendications 1 et 2 **caractérisé en ce que** la cupule (1) présente en débordement de sa face de dessous au moins un ergot anti rotatoire (1F) coopérant avec au moins une entaille (2b) formée dans l'épaisseur de l'embase (2) à partir de son bord périphérique.

4. Dispositif selon la revendication 3 **caractérisé en ce que** la partie lisse (1c) de la cupule (1) présente une section transversale dégressive en direction de l'embase en forme de tronc de cône.

5. Dispositif selon la revendication 3 **caractérisé en ce que** la cupule (1) présente, à partir de sa face d'appui avec l'embase (2), un chambrage présentant des formes de clipage aptes à coopérer avec des formes complémentaires que présente une partie formée en débordement de ladite embase.

6. Dispositif selon l'une quelconque des revendications 1 à 5 **caractérisé en ce que** l'embase (2) présente un plot d'encrage et de stabilité (2c).

7. Dispositif selon la revendication 6 **caractérisé en ce que** le plot (2c) présente au moins une fente (2c1) pour créer un effet de press-fit.

## Patentansprüche

1. Glenoid-Implantat, umfassend eine Cupula (1), die dazu bestimmt ist, mit einem prothetischen Humeruskopf zusammenzuwirken, wobei die genannte Cupula (1) eine allgemeine kreisförmige Form hat und Positionierungs- und Fixierungsanordnungen aufweist, die geeignet sind, ihr Verrasten in der anatomischen Schulterpfanne derart zu erlauben, dass die Aufstütz- und Gleitfläche (1a) der genannten Cupula in die Kontinuität der genannten anatomischen Höhle integriert wird, um eine perfekte Kongruenz mit dem Humeruskopf zu erhalten, **dadurch gekennzeichnet, dass** die Cupula (1) beim Überstehen aus ihrer Peripherie und nur in ihrem oberen Teil verformbare Stabilisierungslamellen (1b) aufweist, die mit der Substantia spongiosa zusammenwirken, wobei der unterer Teil (1c) der Cupula glatt und mit einem Metallsockel in kreisrunder Form (2) gekoppelt ist, wobei der umlaufende Rand des genannten glatten Teils (1c) und der genannte Sockel (2) in einer Fluchtung angeordnet sind, die denselben Durchmesser hat.

2. Vorrichtung gemäß Anspruch 1, **dadurch gekennzeichnet, dass** die Lamellen (1b) in Richtung von oben derart ausgerichtet sind, dass der Einbau und die Verankerung nach dem Einbau begünstigt werden.

3. Vorrichtung gemäß einem den Ansprüchen 1 und 2, **dadurch gekennzeichnet, dass** die Cupula (1) von ihrer unteren Seite wenigstens eine Antirotationsnocke (1F) aufweist, die mit wenigstens einem Einschnitt (2b) zusammenwirkt, der in der Dicke des Sockels (2) ab ihrem umlaufenden Rand gebildet ist.

4. Vorrichtung gemäß Anspruch 3, **dadurch gekennzeichnet, dass** der glatte Teil (1c) der Cupula (1) einen degressiven Querschnitt in Richtung des Sockels in Kegelstumpfform aufweist.

5. Vorrichtung gemäß Anspruch 3, **dadurch gekennzeichnet, dass** die Cupula (1) ab ihrer Aufstützseite auf dem Sockel (2) eine Versenkung aufweist, die Verklippungsformen aufweist, die geeignet sind, mit komplementären Formen zusammenzuwirken, die ein im Überstand des genannten Sockels gebildeter Teil aufweist.

6. Vorrichtung gemäß einem den Ansprüchen 1 bis 5, **dadurch gekennzeichnet, dass** die Basis (2) ein Verankerungs- und Stabilitätsplättchen (2c) aufweist.

7. Vorrichtung gemäß Anspruch 6, **dadurch gekennzeichnet, dass** das Plättchen (2c) wenigstens einen Schlitz (2c1) aufweist, um eine Press-Fit-Wirkung zu erzielen.

## Claims

1. Glenoid implant that includes a cup (1) that is meant to work in conjunction with a prosthetic humeral head, the aforementioned cup (1) is roughly circular in shape, and has fixing and positioning means that enable it to be installed in the anatomical glenoid cavity, such that the supporting and sliding surface (1a) of the aforementioned cup maintains the continuity of the aforementioned anatomical cavity, and aligns perfectly with the humeral head, **characterised by** the fact that protruding from the rim of the cup (1), and only on its upper part, are deformable stabilisation strips (1b) that work in conjunction with the spongiosum, the lower part (1c) of the cup is smooth, and is coupled with a circular metallic base (2), the peripheral edge of the aforementioned smooth part (1c) and the aforementioned base (2) have the same diameter, and are arranged in alignment.

2. The device according to claim 1, **characterised by** the fact that the strips (1b) are oriented upwards so as to facilitate implantation and post-implantation anchoring.

3. The device according to either one of claims 1 and 2, **characterised by** the fact that the cup (1) has, protruding from its underside, at least one anti-rotation pin (1F) that works in conjunction with at least one notch (2b) made in the base (2), starting from its peripheral edge.

4. The device according to claim 3, **characterised by** the fact that the smooth part (1c) of the cup (1) has a decreasing cross-section towards the base, in the shape of a truncated cone.

5. The device according to claim 3, **characterised by** the fact that the cup (1) has, starting from the supporting face that is in contact with the base (2), a recess with clipping shapes that can work in conjunction with complementary shapes located on a part that protrudes from the aforementioned base.

6. The device according to any one of claims 1 to 5, **characterised by** the fact that the base (2) has an anchoring and stability stud (2c).

7. The device according to claim 6, **characterised by** the fact that the stud (2c) has at least one slot (2c1), in order to create a press-fit effect.
